# EUROPÄISCHE PATENTSCHRIFT

(11) **EP 1 889 578 B1**
(45) Veröffentlichungstag und Bekanntmachung des Hinweises auf die Patenterteilung: **01.12.2010**
(21) Anmeldenummer: 07015244.2
(22) Anmeldetag: 03.08.2007
(51) Int. Cl.: A61B 17/28

(54) **Medizinisches Rohrschaftinstrument**
Medical tube shaft instrument
Instrument médical à tige cylindrique

(30) Priorität: 17.08.2006 DE 102006038517
(43) Veröffentlichungstag der Anmeldung: 20.02.2008
(73) Patentinhaber: Karl Storz GmbH & Co. KG, 78532 Tuttlingen (DE)
(72) Erfinder: Renger, Uwe, 78247 Hilzingen (DE); Blocher, Martin, 78532 Tuttlingen (DE); Bacher, Uwe, 78532 Tuttlingen (DE)
(74) Vertreter: Hofmeister, Frank

(56) Entgegenhaltungen:
- DE-A1- 4 307 539
- DE-A1- 19 930 426
- DE-A1- 19 948 031

## Beschreibung

Die Erfindung betrifft ein medizinisches Rohrschaftinstrument mit einem hohlen Schaft, einer am proximalem Ende des Schaftes angeordneten, mit mindestens zwei Griffteilen versehenen Handhabe und mindestens einer in dem hohlen Schaft gelagerten Zug-/Schubstange, an deren distalem Ende ein aus mindestens zwei Maulteilen bestehendes Werkzeug angeordnet ist, wobei die Zug-/Schubstange zum Öffnen und Schließen mindestens eines Maulteils des Werkzeugs mit mindestens einem verschwenkbaren Griffteil der Handhabe koppelbar ist und wobei der hohle Schaft und die Handhabe über einen Kopplungsmechanismus lösbar miteinander verbindbar sind, der als kombinierte Klemm- und Rastvorrichtung ausgebildet ist und zur Ausbildung der Klemmvorrichtung des Kopplungsmechanismus das proximale Ende des hohlen Schaftes konusförmig ausgebildet ist und in der Handhabe eine distalseitig offene und in Axialrichtung verlaufende Aufnahmebohrung zur Aufnahme des proximalen Endes des hohlen Schaftes ausgebildet ist, wobei die Aufnahmebohrurig einen sich entsprechend verjüngenden Gegenkonus aufweist.

Derartige medizinische Rohrschaftinstrumente finden beispielsweise in der Ausbildung als Nadelhalter Verwendung in der endoskopischen Chirurgie. Aufgrund steigender hygienischer Anforderungen wird immer häufiger gefordert, dass insbesondere Hohlräume, wie beispielsweise hohle Schäfte; aufweisende Rohrschaftinstrumente zumindest teilweise zerlegbar ausgebildet sind, um diese einer gründlichen Reinigung und Sterilisation, vorzugsweise Dampfsterilisation, unterziehen zu können.

Ein gattungsgemäßes medizinisches Instrument ist beispielsweise aus der DE 199 30 426 A1 bekannt. Bei diesem bekannten medizinischen Instrument ist der Kopplungsmechanismus als Rast-/Klemmkopplung in einer Baueinheit ausgebildet, die über als Kugeln ausgebildete Rast-/Klemmelemente die Kopplung zwischen dem Schaft und der Handhabe bewirkt. Zwar gewährleistet dieser Kopplungsmechanismus eine zuverlässige Kopplung der miteinander zu verbindenden Bauteile, jedoch ist die bekannte Konstruktion mit den federbelasteten Kugeln und den zugehörigen, mit unterschiedlichen Winkeln ausgebildeten Anlaufschrägen für die Kugeln sehr aufwändig.

Weiterhin ist aus der DE 43 07 539 A1 ein als medizinische Zange ausgebildetes medizinisches Rohrschaftinstrument bekannt. Dieses bekannte Rohrschaftinstrument lässt sich zum Reinigen und Sterilisieren in drei Hauptgruppen zerlegen, die Zug-/Schubstange, den hohlen Schaft sowie die Handhabe. Der Kopplungsmechanismus zum Verbinden von hohlem Schaft und Handhabe ist bei dieser Konstruktion als Rastverbindung ausgebildet. Rastverbindungen an sich weisen jedoch in der Regel ein gewisses Bewegungsspiel auf, wenn sie nicht mit äußerst engen Toleranzen gefertigt werden, die wiederum die Fertigungskosten erhöhen und die Handhabung bei der Montage erschweren.

Davon ausgehend liegt der Erfindung die **Aufgabe** zugrunde, ein medizinisches Rohrschaftinstrument derart weiterzubilden, dass der hohle Schaft und die Handhabe über einen weitestgehend spielfrei arbeitenden Kopplungsmechanismus lösbar miteinander verbindbar sind.

Die **Lösung** dieser Aufgabenstellung ist erfindungsgemäß **dadurch gekennzeichnet, dass** der Gegenkonus zur Aufnahme des proximalen Endes des hohlen Schaftes in einer in Axialrichtung verschiebbar in der Aufnahmebohrung gelagerten Hülse ausgebildet ist, wobei die Hülse gegen die Kraft eines Federelements axialverschiebbar in der Aufnahmebohrung gelagert ist und die Hülse über das Federelement in die die Kopplung zwischen hohlem Schaft und Handhabe freigebende Montagestellung vorgespannt ist.

Durch die Kombination von Klemm- und Rastverbindung werden auch die Vorteile beider Verbindungstechniken kombiniert, nämlich die einfache Montage und Demontage einer Rastverbindung und die Spielfreiheit einer Klemmverbindung.

Die Ausbildung von konusförmige Schaftende und Gegenkonus in der Aufnahmebohrung ermöglicht eine automatische und nahezu spielfreie Kopplung der miteinander zu verbindenden Bauteile, die einerseits einfach und kostengünstig zu fertigen ist und andererseits einfach und schnell montierbar und wieder demontierbar ist.

Die Ausbildung des Gegenkonus in einer in die Aufnahmebohrung einzusetzenden Hülse vereinfacht die Fertigung des Gegenkonus und ermöglicht auch die Anpassung an unterschiedliche Konuswinkel des Schaftendes.

Die Axialverschiebbarkeit der Hülse vereinfacht die Ausrichtung des Schaftendes in Bezug auf die Rastvorrichtung, wodurch auch Fertigungstoleranzen einfach ausgleichbar sind, da nur der Gegenkonus der Hülse mit genauen Toleranzen zu fertigen ist, um die klemmwirkung der gegenseitigen Konusflächen zu erzielen, hochgenaue Toleranzen hinsichtlich der Längenausbildung des hohlen Schaftes aber nicht erforderlich sind.

Zur Ausbildung der Rastvorrichtung wird mit der Erfindung vorgeschlagen, dass in der Handhabe ein im Wesentlichen quer zur Aufnahmebohrung verlagerbares Rastelement angeordnet ist.

Gemäß einer bevorzugten Ausführungsform der Erfindung wird vorgeschlagen, dass das Rastelement aus einem Sperrglied sowie einem mit dem Sperrglied gekoppelten Freigabeknopf besteht, wobei das Rastelement vorteilhafterweise über ein Federelement in Richtung auf die Raststellung vorgespannt ist. Das Vorspannen des Rastelements in Richtung der Sperrstellung hat den Vorteil, dass es automatisch zu der gewünschten Verrastung kommt, sobald die miteinander zu koppelnden Bauteile sich in der richtigen Position zum Rastelement befinden.

Um ein sicheres Festlegen des hohlen Schaftes mittels der Rastvorrichtung zu gewährleisten, ist im proximalen Ende des hohlen Schaftes mindestens eine Ausnehmung zur verrastenden Aufnahme des Rastelements ausgebildet. Vorteilhafterweise ist sowohl im proximalen Ende des hohlen Schaftes als auch in der Hülse mindestens eine Ausnehmung zur verrastenden Aufnahme des Rastelements ausgebildet, um über nur ein Rastelement das konusförmige Schaftende und den Gegenkonus fixieren zu können.

Das lagegerechte Einsetzen des Schaftendes in die Aufnahmebohrung, insbesondere hinsichtlich der Ausrichtung zur Rastvorrichtung, kann erfindungsgemäß dadurch erleichtert werden, dass in der Handhabe mindestens ein Führungselement angeordnet ist, über das das proximale Ende des hohlen Schaftes in der Aufnahmebohrung ausrichtbar ist, wobei das Führungselement vorzugsweise als Stift oder beispielsweise in die Handhabe einschraubbare Madenschraube ausgebildet ist.

Gemäß einer praktischen Ausführungsform der Erfindung ist am proximalen Ende des hohlen Schaftes mindestens eine Führungsnut zur Aufnahme des Führungselements ausgebildet ist. Vorzugsweise sind am proximalen Ende des hohlen Schaftes zwei einander gegenüberliegende Führungsnuten zur Aufnahme des Führungselements ausgebildet. Die Verwendung von zwei einander gegenüberliegende Führungsnuten zur Aufnahme des Führungselements hat den Vorteil, dass auch bei nur einem aufzunehmendem Führungselement die beiden miteinander zu koppelnden Bauteile nicht nur in einer einzigen Stellung der Bauteile zueinander miteinander koppelbar sind, wodurch die Montage vereinfacht wird.

Schließlich wird mit der Erfindung vorgeschlagen, dass das Führungselement einen Anschlag für die Hülse bildet und so verhindert, dass die Hülse nach dem Lösen der Rastverbindung versehentlich zusammen mit dem hohlen Schaft aus der Aufnahmebohrung herausgezogen wird.

Weitere Merkmale und Vorteile der Erfindung ergeben sich anhand der zugehörigen Zeichnung, in der ein Ausführungsbeispiel eines erfindungsgemäßen medizinischen Rohrschaftinstruments nur beispielhaft dargestellt ist. In der Zeichnung zeigt:
- Fig. 1: eine schematische Seitenansicht eines erfindungsgemäßen Rohrschaftinstruments;
- Fig. 2: eine schematische Seitenansicht des Instruments gemäß Fig. 1 im zerlegten Zustand;
- Fig. 3a: eine vergrößerte ausschnittweise schematische Schnittdarstellung des Details IIIa gemäß Fig. 1 in Seitenansicht während der Montage;
- Fig. 3b: eine Ansicht gemäß Fig. 3a, jedoch aus der Draufsicht;
- Fig. 4a: eine Ansicht gemäß Fig. 3a, jedoch die verrastete Arbeitsstellung darstellend;
- Fig. 4b: eine Ansicht gemäß Fig. 4a, jedoch aus der Draufsicht und
- Fig. 5: einen schematischen Schnitt entlang der Linie V-V gemäß Fig. 3a, jedoch ausschließlich das Rastelement darstellend.

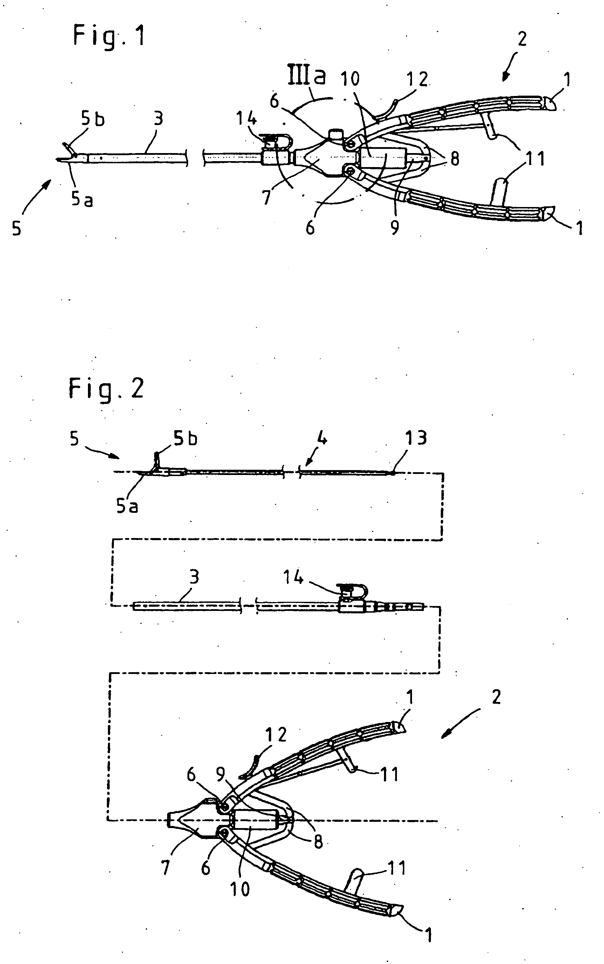

Das in den Abbildungen Fig. 1 und 2 dargestellte, als Nadelhalter ausgebildete medizinische Rohrschaftinstrument besteht im Wesentlichen aus einer mit zwei Griffteilen 1 versehenen Handhabe 2, einem hohlen Schaft 3 sowie einer in den hohlen Schaft 3 einsetzbaren Zug-/Schubstange 4, an deren distalem Ende ein aus zwei Maulteilen 5a und 5b bestehendes Werkzeug 5 angeordnet ist.

Die in Fig. 2 besonders deutlich dargestellten Baugruppen Handhabe 2, hohler Schaft 3 und Zug-/Schubstange 4 sind über Kopplungs- und Rastmechanismen so miteinander koppelbar, dass durch Betätigen der Griffteile 1 der Handhabe 2 die Maulteile 5a und 5b des Werkzeugs 5 zwischen einer offenen und einer geschlossenen Arbeitsstellung verstellbar sind, wobei die vom Benutzer auf die Griffteile 1 der Handhabe 2 aufgebrachten Kräfte über die Zug-/Schubstange 4 auf die Maulteile 5a, 5b des Werkzeugs 5 übertragen werden.

Bei der dargestellten Ausführungsform weist das Werkzeug 5 ein starres Maulteil 5a und ein gegenüber dem starren Maulteil 5a verschwenkbares Maulteil 5b auf. Selbstverständlich ist es auch möglich, beide Maulteile des Werkzeugs 5 als verschwenkbare Maulteile 5b auszubilden.

Wie aus Fig. 1 und 2 ersichtlich, sind bei der dargestellten Ausführungsform beide Griffteile 1 der Handhabe 2 als verschwenkbare Griffteile 1 ausgebildet, die über Anlenkpunkte 6 verschwenkbar an einem Gehäuse 7 der Handhabe 2 gelagert sind. Zur Überführung der Schwenkbewegung der Griffteile 1 in eine reine Axialbewegung der Zug-/Schubstange 4 sowie zur Kraftübertragung der vom Benutzer über die Handhabe 2 eingeleiteten Druckkraft auf die Zug-/Schubstange 4 sind beide Griffteile 1 über jeweils einen Gelenkhebel 8 mit einer Kupplungsstange 9 verbunden, die ihrerseits direkt oder indirekt über einen Kopplungsmechanismus mit der Zug-/Schubstange 4 gekoppelt ist, wobei die Kopplung der Zug-/Schubstange 4 mit der Kupplungsstange 9 und somit mit der Handhabe 2 in dem Kupplungsgehäuse 10 erfolgt.

Die Kopplung der Zug-/Schubstange 4 und somit auch der Maulteile 5a und 5b des Werkzeugs 5 mit den Griffteilen 1 der Handhabe 2 ist so ausgelegt, dass beim Zusammendrücken der Griffteile 1 die Zug-/Schubstange 4 über die Gelenkhebel 8 und die Kupplungsstange 9 in axialer Richtung zum proximalen Ende des Instruments gezogen wird. Diese Axialverschiebung der Zug-/Schubstange 4 zum proximalen Ende des Instruments bewirkt ein Überführen der Maulteile 5a, 5b des Werkzeugs 5 in die geschlossene Arbeitsstellung. In dieser zusammengedrückten Stellung sind die Griffteile 1 über eine Arretiervorrichtung 11 gegeneinander fixierbar, so dass der Benutzer nicht die gesamte Zeit die Druckkraft auf die Griffteile 1 der Handhabe 2 ausüben muss. Über einen Entriegelungsknopf 12, der die Teile der Arretiervorrichtung 11 trennt, ist diese Fixierung wieder aufhebbar.

Alternativ zu der dargestellten Ausführungsform ist es selbstverständlich auch möglich, das Rohrschaftinstrument so auszugestalten, dass das Überführen der Maulteile 5a, 5b des Werkzeugs 5 in die geschlossene Arbeitsstellung über eine Axialverschiebung der Zug-/Schubstange 4 zum distalen Ende des Instruments bewirkt wird.

Vorteilhafterweise sind die Griffteile 1 über ein Federelement in die Offenstellung vorgespannt, das beispielsweise im Kupplungsgehäuse 10 angeordnet sein kann. Sobald der Entriegelungsknopf 12 betätigt wird, schiebt dann dieses Federelement die Zug-/Schubstange 4 in axialer Richtung zum distalen Ende des Instruments, wodurch die Griffteile 1 über die Kupplungsstange 9 und die Gelenkhebel 8 auseinander gedrückt werden. Diese Axialverschiebung der Zug-/Schubstange 4 zum distalen Ende des Instruments bewirkt ein Überführen der Maulteile 5a, 5b des Werkzeugs 5 in die offene Arbeitsstellung.

Alternativ zu der dargestellten Ausführungsform der Handhabe 2 mit zwei verschwenkbaren Griffteilen 1 ist es selbstverständlich auch möglich, nur ein Griffteil 1 verschwenkbar auszubilden, wohingegen das andere Griffteil dann beispielsweise einstückig starr mit dem Gehäuse 7 der Handhabe 2 ausgebildet ist. Bei einer solchen Ausgestaltung ist es möglich, die Zug-/Schubstange 4 direkt mit dem verschwenkbaren Griffteil 1 zu koppeln.

Weiterhin ist es möglich, im Kopplungsbereich der Zug-/Schubstange 4 mit der Handhabe 2 eine Überlastsicherung vorzusehen, die eine zu große Krafteinleitung in die Zug-/Schubstange 4 verhindert. Eine solche Überlastsicherung kann beispielsweise als Überlastfeder ausgebildet im Bereich der Kupplungsstange 9 angeordnet sein. Zum Verbinden der Zug-/Schubstange 4 mit der Handhabe 2 ist am proximalen Ende der Zug-/Schubstange 4 ein Kopplungselement 13 angeordnet, über das die Zug-/Schubstange 4 lösbar mit der Handhabe 2 verbindbar ist.

Auch distalseitig weist der hohle Schaft 3 einen Kopplungs- oder Rastmechanismus auf, um den hohlen Schaft 3 und die in diesen einsetzbare Zug-/Schubstange 4 miteinander koppeln zu können.

Der zur Aufnahme der Zug-/Schubstange 4 dienende hohle Schaft 3 ist über einen Kopplungsmechanismus mit der Handhabe 2 koppelbar, der im Gehäuse 7 der Handhabe 2 angeordnet ist. Bei der dargestellten Ausführungsform des medizinischen Rohrschaftinstruments weist der hohle Schaft 3 weiterhin einen Spülanschluss 14 auf, der einerseits zum Einleiten von Spülflüssigkeit während einer Operation dient und an den andererseits zum Reinigen des hohlen Schafts 3 ein Spülschlauch anschließbar ist.

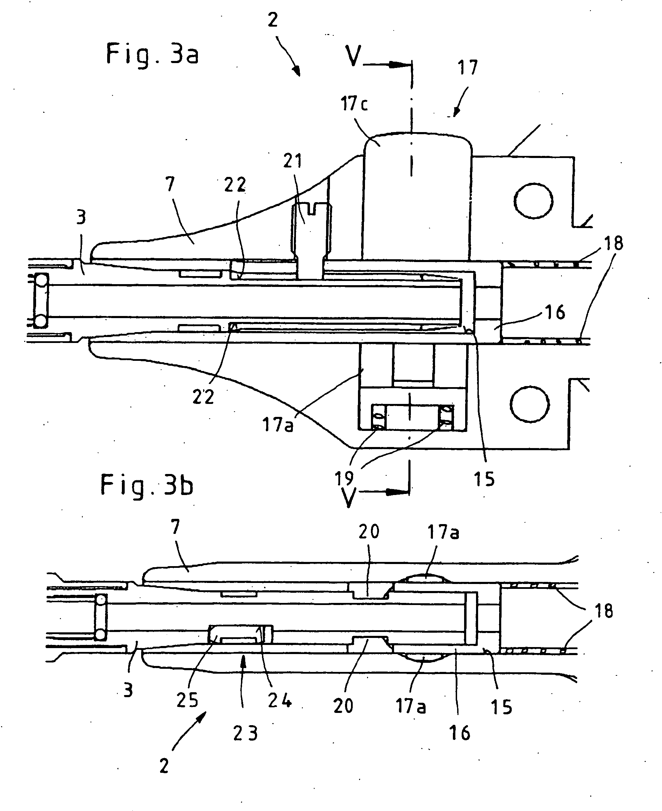

Der Aufbau des Kopplungsmechanismus zum lösbaren Verbinden des hohlen Schafts 3 mit der Handhabe 2 ist den Abbildungen Fig. 3a bis 5 zu entnehmen.

Dieser Kopplungsmechanismus ist als kombinierte Klemm- und Rastvorrichtung ausgebildet, deren wesentliche Bestandteile das konusförmig sich verjüngende proximale Ende des hohlen Schaftes 3, eine in Axialrichtung des Rohrschaftinstruments verlaufend im Gehäuse 7 der Handhabe 2 ausgebildete Aufnahmebohrung 15 sowie eine in der Aufnahmebohrung 15 angeordnete Hülse 16 zur Ausbildung der Klemmvorrichtung und ein quer zur Aufnahmebohrung 15 verlagerbares Rastelement 17 zur Ausbildung der Rastvorrichtung sind.

Um eine im Wesentlichen formschlüssige Klemmverbindung zwischen dem proximalen Ende des hohlen Schaftes 3 und der Hülse 16 zu gewährleisten, ist das Innere der Hülse 16 als ein sich entsprechend dem konusförmigen Ende des hohlen Schaftes 3 verjüngender Gegenkonus ausgebildet.

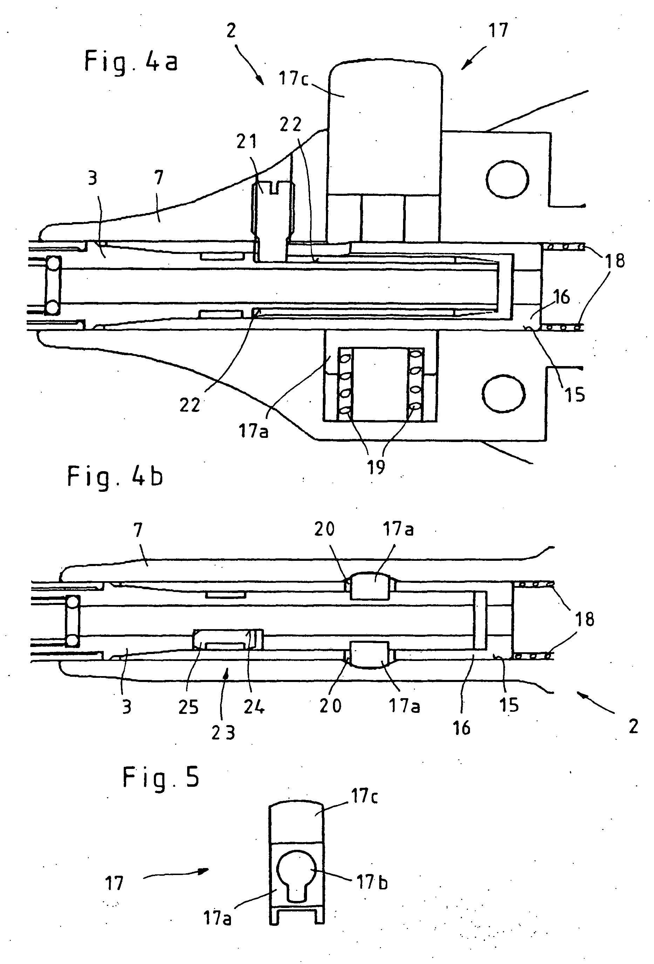

Wie weiterhin aus den Abbildungen Fig. 3a und 4a ersichtlich, ist die Hülse 16 gegen die Kraft eines Federelements 18 axialverschiebbar so in der Aufnahmebohrung 15 angeordnet, dass die Hülse 16 über das Federelement 18 in distale Richtung und somit in Richtung der Montagestellung vorgespannt ist, in der sich die Hülse 16 in der ungekoppelten Stellung befindet.

Das die Rastvorrichtung bildende Rastelement 17 besteht bei der dargestellten Ausführungsform, wie insbesondere aus Fig. 5 ersichtlich, aus einem Sperrglied 17a, welches eine im Querschnitt schlüssellochförmige Durchgangsbohrung 17b zur Aufnahme der mit dem proximalen Ende des hohlen Schaftes 3 verklemmten Hülse 16 aufweist, sowie einem mit dem Sperrglied 17a in Wirkverbindung stehenden Freigabeknopf 17c.

Wie weiterhin aus den Abbildungen Fig. 3a und 4a ersichtlich, ist das Rastelement 17 gegen die Kraft eines Federelements 19 quer zur Aufnahmebohrung 15 verschiebbar so im Gehäuse 7 der Handhabe 2 angeordnet, dass das Sperrglied 17a über das Federelement 19 in Richtung der Raststellung vorgespannt ist, in der das Sperrglied 17a das proximale Ende des hohlen Schaftes 3 verrastend ergreift.

Das Festlegen des hohlen Schaftes 3 mittels des Sperrglieds 17a erfolgt bei der dargestellten Ausführungsform über jeweils zwei im proximalen Ende des hohlen Schaftes 3 sowie deckungsgleich in der Hülse 16 ausgebildete Ausnehmungen 20, in die das Sperrglied 17a des Rastelements 17 arretierend eingreift. Unter Hinzuziehung der Abbildung Fig. 5 lässt sich das Verrasten des hohlen Schaftes 3 mit dem Sperrglied 17a des Rastelements 17 so verdeutlichen, dass die mit dem Schaftende verklemmte Hülse 16 in der noch nicht verrasteten Stellung im Bereich des oberen runden Teils der Durchgangsbohrung 17b des Sperrglieds 17a gelagert ist. Sobald die in der Hülse 16 sowie dem Schaftende ausgebildeten Ausnehmungen 20 in die Durchgangsbohrung 17b eintreten, wird das Sperrglied 17a über das Federelement 19 nach oben gedrückt, wodurch die mit dem Schaftende verklemmte Hülse 16 nunmehr in den schmalen unteren Bereich der Durchgangsbohrung 17b des Sperrglieds 17a eintritt. In dieser in Fig. 4a und 4b dargestellten verrasteten Arbeitsstellung sind sowohl die Hülse 16 als auch der hohle Schaft 3 gegen axiales Verschieben in der Handhabe 2 fixiert.

Um das lagegerechte Einsetzen des Schaftendes in die Aufnahmebohrung 15 zu erleichtern, insbesondere hinsichtlich der Ausrichtung zur Rastvorrichtung, ist im Gehäuse 7 der Handhabe 2 weiterhin ein Führungselement 21 angeordnet, über das das proximale Ende des hohlen Schaftes 3 in der Aufnahmebohrung 15 ausrichtbar ist. Bei der dargestellten Ausführungsform ist das Führungselement 21 als in das Gehäuse 7 der Handhabe 2 einschraubbare Madenschraube ausgebildet, die in eine am proximalen Ende des hohlen Schaftes 3 ausgebildete Führungsnut 22 eingreift. Statt nur eine Führungsnut 22 zu verwenden ist es auch möglich, mehrere Führungsnuten 22 am proximalen Ende des hohlen Schaftes 3 auszubilden.

Wie aus Fig. 3a und 4a ersichtlich, sind bei der dargestellten Ausführungsform am proximalen Ende des hohlen Schaftes 3 zwei einander gegenüberliegende Führungsnuten 22 zur Aufnahme des Führungselements 21 ausgebildet. Die Verwendung von zwei einander gegenüberliegende Führungsnuten 22 zur Aufnahme des Führungselements 21 hat den Vorteil, dass auch bei nur einem aufzunehmendem Führungselement 21 die beiden miteinander zu koppelnden Bauteile nicht nur in einer einzigen Stellung der Bauteile zueinander miteinander koppelbar sind, wodurch die Montage vereinfacht wird.

Alternativ zu der dargestellten Ausführungsform ist es selbstverständlich auch möglich, die Führungsnut zur Aufnahme des Führungselements 21 als umlaufende Nut auszubilden, wenn sich die Zug-/Schubstange 4 des medizinischen Instruments um ihre Längsachse drehen darf.

Das Zusammensetzen des dargestellten Rohrschaftinstruments und insbesondere des Kopplungsmechanismus zum Verbinden des hohlen Schaftes 3 mit der Handhabe wird nachfolgend anhand der Abbildungen Fig. 3a bis 5 beschrieben.

Im ersten Montageschritt werden die Zug-/Schubstange 4 und der hohle Schaft 3 miteinander gekoppelt, wobei, um ein versehentliches Lösen der Kopplung zwischen der Zug-/Schubstange 4 und dem hohlen Schaft 3 zu vermeiden, die dargestellte Ausführungsform im Bereich des in der Handhabe 2 gelagerten proximalen Endes der Zug-/Schubstange 4 und des hohlen Schaftes 3 eine die Verlagerbarkeit der Zug-/Schubstange 4 innerhalb des hohlen Schaftes 3 limitierende Begrenzungsvorrichtung 23 aufweist.

Wie aus Fig. 3b und 4b ersichtlich, besteht die dargestellte Begrenzungsvorrichtung 23 aus zwei an der Zug-/Schubstange 4 ausgebildeten, einander gegenüberliegend angeordneten Anlageflächen 24, die als Ausnehmungen in der Zug-/Schubstange 4 ausgebildet sind, sowie aus einem in einer Ausnehmung im hohlen Schaft 3 gelagerten Anlageelement 25, das im montierten Zustand von Zug-/Schubstange 4 und der hohle Schaft 3 im Wesentlichen formschlüssig an einer Anlagefläche 24 der Zug-/Schubstange 4 anliegt.

Diese nur im fertig montierten Zustand des Rohrschaftinstruments wirksame Begrenzungsvorrichtung 23 erlaubt einen Axialhub der Zug-/Schubstange 4 nur innerhalb der begrenzten Wegstrecke, die notwendig ist, um die Maulteile 5a, 5b des Werkzeugs 5 zwischen der in Fig. 1 dargestellten offenen Arbeitsstellung und der geschlossenen Arbeitsstellung zu verschwenken. Ein Lösen der Kopplung zwischen der Zug-/Schubstange 4 und dem hohlen Schaft 3 im montierten Zustand wird durch die Begrenzungsvorrichtung 23 unmöglich gemacht, da das Anlageelement 25 der Begrenzungsvorrichtung 23 im mit der Handhabe 2 gekoppelten Zustand des hohlen Schaftes 3 nicht radial nach außen aus der Ausnehmung in der Zug-/Schubstange 4 heraustreten kann. Aufgrund der Kopplung der Axialverlagerung der Zug-/Schubstange 4 mit dem Verschwenkwinkel der Griffteile 1 der Handhabe 2 bewirkt somit die den Axialhub der Zug-/Schubstange 4 beschränkende Begrenzungsvorrichtung 23 gleichzeitig eine Limitierung des Verschwenkwinkel der Griffteile 1 im fertig montierten Zustand.

Im nachfolgenden zweiten Montageschritt wird das konusförmige proximale Ende des hohlen Schaftes 3 in die im Gehäuse 7 der Handhabe 2 ausgebildete Aufnahmebohrung 15 eingeschoben, bis dieses Aufnahme in der in der Aufnahmebohrung 15 angeordneten Hülse 16 findet, wobei der hohle Schaft 3 bezüglich Drehung um seine Längsachse durch das als Madenschraube ausgebildete Führungselement 21 in der Aufnahmebohrung 15 sowie der Hülse 16 bezüglich der Rastvorrichtung ausgerichtet wird. Sobald nämlich das Führungselement 21 in eine der Führungsnuten 22 eingreift, ist das Schaftende lagegerecht positioniert und der hohle Schaft 3 kann nicht mehr um seine Längsachse verdreht werden.

Jetzt wird das konusförmige Schaftende weiter in die Hülse 16 eingedrückt, bis sich das Schaftende mit dem Gegenkonus der Hülse 16 verklemmt. Durch weiter Druckkraft über den hohlen Schaft 3 in proximaler Richtung wird die mit dem Schaftende verklemmte Hülse 16 gegen die Kraft des Federelements 18 weiter in proximaler Richtung in die Aufnahmebohrung 15 hineingedrückt, bis das Rastelement 17 die Axialverschiebung der Hülse 16 verrastend beendet.

Parallel zu dieser Verrastung des proximalen Endes des hohlen Schaftes 3 mit der Handhabe 2 oder auch nachfolgend zu dieser Verrastung erfolgt die Kopplung der Zug-/Schubstange 4 mit den Griffteilen 1 der Handhabe 2, um über das Verschwenken der Griffteile 1 eine Axialverschiebung der Zug-/Schubstange 4 bewirken zu können, die wiederum das Verstellen der Maulteile 5a und 5b des Werkzeugs 5 bewirkt.

Sobald die mit dem Schaftende verklemmte Hülse 16 soweit in die Aufnahmebohrung 15 eingeschoben worden ist, dass die in der Hülse 16 sowie dem hohlen Schaft 3 ausgebildeten Ausnehmungen 20 in Höhe des Rastelements 17 zu liegen kommen, drückt das Federelement 19 das Sperrglied 17a des Rastelements 17 in diese Ausnehmungen und blockiert so jegliche weitere Axialverschiebung von Hülse 16 und hohlem Schaft 3. Durch die Kombination von Klemmverbindung und Rastverbindung gewährleistet dieser Kopplungsmechanismus eine größtmögliche Spielfreiheit der Verbindung.

Die Demontage erfolgt dann in genau umgekehrter Reihenfolge der Montageschritte über das Lösen der Klemmrastung zwischen dem hohlen Schaft 3 und der Handhabe 2, das Lösen der Verbindung der Zug-/Schubstange 4 mit den Griffteilen 1 der Handhabe 2, bis hin zum Entkoppeln des hohlen Schaftes 3 von der Zug-/Schubstange 4.

Zum Lösen der Klemmrastung zwischen dem hohlen Schaft 3 und der Handhabe 2 wird zunächst die Rastverbindung durch Abwärtsdrücken des Freigabeknopfs 17c aufgehoben. Durch das Betätigen des Freigabeknopfs 17c gibt das Sperrglied 17a die Ausnehmungen 20 der Hülse 16 sowie des hohlen Schaftes 3 wieder frei. Sobald diese Verrastung nicht mehr besteht, wird die mit dem Schaftende verklemmte Hülse 16 über das Federelement 18 in distale Richtung aus der Aufnahmebohrung 15 gedrückt.

Das Herausdrücken der Hülse 16 aus der Aufnahmebohrung 15 über das Federelement 18 bewirkt gleichzeitig eine Verlagerung des hohlen Schaftes 3 mitsamt der in dem hohlen Schaft 3 gelagerten Zug-/Schubstange 4 in distaler Richtung, Diese Bewegung in distaler Richtung wird zusätzlich durch die mit den Griffteilen 1 gekoppelte Kupplungsstange 9 unterstützt, die durch die über ein Federelement in die Offenstellung vorgespannten Griffteile 1 in distale Richtung gedrückt wird, sobald die Klemmrastung zwischen dem hohlen Schaft 3 und der Handhabe 2 gelöst wird.

Nachfolgend zu der Axialverschiebung des hohlen Schaftes 3 mitsamt der in dem hohlen Schaft 3 gelagerten Zug-/Schubstange 4 in distaler Richtung tritt die Begrenzungsvorrichtung 23 außer Kraft und gibt die Kopplung zwischen dem hohlen Schaft 3 und der Zug-/Schubstange 4 wieder frei, da das nunmehr nicht mehr in der Handhabe 2 gelagerte Anlageelement 25 jetzt in radialer Richtung nach außen aus der Ausnehmung der Zug-/Schubstange 4 heraustreten kann, sobald eine axiale Schubkraft in distaler Richtung aus das proximale Ende der Zug-/Schubstange 4 ausgeübt wird.

Diese Axialverschiebung der Hülse 16 wird durch das Führungselement 21 begrenzt, das einen Anschlag für die Axialverschiebbarkeit der Hülse in distaler Richtung bildet. Jetzt kann der hohle Schaft 3 durch Ziehen in distale Richtung aus der Klemmverbindung mit der Hülse 16 herausgezogen werden.

### Bezugszeichenliste

- 1: Griffteil
- 2: Handhabe
- 3: hohler Schaft
- 4: Zug-/Schubstange
- 5: Werkzeug
- 5a: starres Maulteil
- 5b: verschwenkbares Maulteil
- 6: Anlenkpunkt
- 7: Gehäuse
- 8: Gelenkhebel
- 9: Kupplungsstange
- 10: Kupplungsgehäuse
- 11: Arretiervorrichtung
- 12: Entriegelungsknopf
- 13: Kopplungselement
- 14: Spülanschluss
- 15: Aufnahmebohrung
- 16: Hülse
- 17: Rastelement
- 17a: Sperrglied
- 17b: Durchgangsbohrung
- 17c: Freigabeknopf
- 18: Federelement
- 19: Federelement
- 20: Ausnehmung
- 21: Führungselement
- 22: Führungsnut
- 23: Begrenzungsvorrichtung
- 24: Anlagefläche
- 25: Anlageelement

## Patentansprüche

1. Medizinisches Rohrschaftinstrument mit einem hohlen Schaft (3), einer am proximalem Ende des Schaftes (3) angeordneten, mit mindestens zwei Griffteilen (1) versehenen Handhabe (2) und mindestens einer in dem hohlen Schaft (3) gelagerten Zug-/Schubstange (4), an deren distalem Ende ein aus mindestens zwei Maulteilen (5a, 5b) bestehendes Werkzeug (5) angeordnet ist, wobei die Zug-/Schubstange (4) zum Öffnen und Schießen mindestens eines Maulteils (5b) des Werkzeugs (5) mit mindestens einem verschwenkbaren Griffteil (1) der Handhabe (2) koppelbar ist und wobei der hohle Schaft (3) und die Handhabe (2) über einen Kopplungsmechanismus lösbar miteinander verbindbar sind, wobei der Kopplungsmechanismus als kombinierte Klemm- und Rastvorrichtung ausgebildet ist und zur Ausbildung der Klemmvorrichtung das proximale Ende des hohlen Schaftes (3) konusförmig ausgebildet ist und in der Handhabe (2) eine distalseitig offene und in Axialrichtung verlaufende Aufnahmebohrung (15) zur Aufnahme des proximalen Endes des hohlen Schaftes (3) ausgebildet ist, wobei die Aufnahmebohrung (15) einen sich entsprechend verjüngenden Gegenkonus aufweist,
**dadurch gekennzeichnet,**
**dass** der Gegenkonus zur Aufnahme des proximalen Endes des hohlen Schaftes (3) in einer in Axialrichtung verschiebbar in der Aufnahmebohrung (15) gelagerten Hülse (16) ausgebildet ist, wobei die Hülse (16) gegen die Kraft eines Federelements (18) axialverschiebbar in der Aufnahmebohrung (15) gelagert ist und die Hülse (16) über das Federelement (18) in die die Kopplung zwischen hohlem Schaft (3) und Handhabe (2) freigebende Montagestellung vorgespannt ist.

2. Medizinisches Rohrschaftinstrument nach Anspruch 1, **dadurch gekennzeichnet, dass** zur Ausbildung der Rastvorrichtung in der Handhabe (2) ein im Wesentlichen quer zur Aufnahmebohrung (15) verlagerbares Rastelement (17) angeordnet ist.

3. Medizinisches Rohrschaftinstrument nach Anspruch 2, **dadurch gekennzeichnet, dass** das Rastelement (17) aus einem Sperrglied (17a) sowie einem mit dem Sperrglied (17a) gekoppelten Freigabeknopf (17c) besteht.

4. Medizinisches Rohrschaftinstrument nach Anspruch 2 oder 3, **dadurch gekennzeichnet, dass** das Rastelement (17) über ein Federelement (19) in Richtung auf die Raststellung vorgespannt ist.

5. Medizinisches Rohrschaftinstrument nach einem der Ansprüche 2 bis 4, **dadurch gekennzeichnet, dass** im proximalen Ende des hohlen Schaftes (3) mindestens eine Ausnehmung (20) zur verrastenden Aufnahme des Rastelements (17) ausgebildet ist.

6. Medizinisches Rohrschaftinstrument nach Anspruch 5, **dadurch gekennzeichnet, dass** sowohl im proximalen Ende des hohlen Schaftes (3) als auch in der Hülse (16) mindestens eine Ausnehmung (20) zur verrastenden Aufnahme des Rastelements (17) ausgebildet ist.

7. Medizinisches Rohrschaftinstrument nach einem der Ansprüche 1 bis 6, **dadurch gekennzeichnet, dass** in der Handhabe (2) mindestens Führungselement (21) angeordnet ist, über das das proximale Ende des hohlen Schaftes (3) in der Aufnahmebohrung (15) ausrichtbar ist.

8. Medizinisches Rohrschaftinstrument nach Anspruch 7, **dadurch gekennzeichnet, dass** das Führungselement (21) als Madenschraube ausgebildet ist.

9. Medizinisches Rohrschaftinstrument nach Anspruch 7 oder 8, **dadurch gekennzeichnet, dass** am proximalen Ende des hohlen Schaftes (3) mindestens eine Führungsnut (22) zur Aufnahme des Führungselements (21) ausgebildet ist.

10. Medizinisches Rohrschaftinstrument nach Anspruch 9, **dadurch gekennzeichnet, dass** am proximalen Ende des hohlen Schaftes (3) zwei einander gegenüberliegende Führungsnuten (22) zur Aufnahme des Führungselements (21) ausgebildet sind.

11. Medizinisches Rohrschaftinstrument nach einem der Ansprüche 7 bis 10, **dadurch gekennzeichnet, dass** das Führungselement (21) einen Anschlag für die Hülse (16) bildet.

## Claims

1. A medical tube shaft instrument with a hollow shaft (3), a handle (2) arranged at the proximal end of the hollow shaft (3) and provided with at least two grip parts (1), and with at least one pull/push rod (4) supported in the hollow shaft (3), at the distal end of which pull/push rod a tool (5) is arranged which consists of at least two jaw parts (5a, 5b), wherein the pull/push rod (4) for opening and closing of at least one jaw part (5b) of the tool (5) can be coupled with at least one pivotable grip part (1) of the handle (2), and wherein the hollow shaft (3) and the handle (2) can be detachably connected to each other via a coupling mechanism, wherein the coupling mechanism is formed as a combined clamping and locking device, and for forming the clamping device, the proximal end of the hollow shaft (3) has a conical shape, and in the handle (2), a receiving bore (15) is formed which is distally open and runs in axial direction for receiving the proximal end of the hollow shaft (3), wherein the receiving bore (15) has a correspondingly tapered counter cone,
**characterized in**
**that** for receiving the proximal end of the hollow shaft (3), the counter cone is formed in a sleeve (16) which is supported in the receiving bore (15) and movable in axial direction, wherein the sleeve (16) is supported in the receiving bore (15) and axially movable against the force of a spring element (18), and the sleeve (16) is pretensioned via the spring element (18) into the assembly position which releases the coupling between hollow shaft (3) and handle (2).

2. The medical tube shaft element according to claim 1, **characterized in that** for forming the locking device, a locking element (17) is arranged in the handle (2), which locking device is displaceable substantially transverse to the receiving bore (15).

3. The medical tube shaft element according to claim 2, **characterized in that** the locking element (17) consists of a blocking member (17a) and a release button (17c) which is coupled with the blocking member (17a).

4. The medical tube shaft element according to claim 2 or claim 3, **characterized in that** the locking element (17) is pretensioned via a spring element (19) toward the locking position.

5. The medical tube shaft element according to any one of the claims 2 to 4, **characterized in that** in the proximal end of the hollow shaft (3) at least one recess (20) is formed for receiving the locking element (17) in a locking manner.

6. The medical tube shaft element according to claim 5, **characterized in that** in the proximal end of the hollow shaft (3) as well as in the sleeve (16) at least one recess (20) is formed for receiving the locking element (17) in a locking manner.

7. The medical tube shaft element according to any one of the claims 1 to 6, **characterized in that** in the handle (2) at least one guide element (21) is arranged by means of which the proximal end of the hollow shaft (3) can be aligned in the receiving bore (15).

8. The medical tube shaft element according to claim 7, **characterized in that** the guide element (21) is formed as grub screw.

9. The medical tube shaft element according to claim 7 or claim 8, **characterized in that** at the proximal end of the hollow shaft (3) at least one guide groove (22) for receiving the guide element (21) is formed.

10. The medical tube shaft element according to claim 9, **characterized in that** at the proximal end of the hollow shaft (3) two opposing guide grooves (22) for receiving the guide element (21) are formed.

11. The medical tube shaft element according to any one of the claims 7 to 10, **characterized in that** the guide element (21) forms a limit stop for the sleeve (16).

## Revendications

1. Instrument médical à corps allongé tubulaire comprenant un corps allongé creux (3), une poignée de manoeuvre (2) agencée à l'extrémité proximale du corps allongé creux (3) et dotée d'au moins deux branches de préhension (1), et au moins une tige de traction/poussée (4) montée dans le corps allongé creux (3) et à l'extrémité distale de laquelle est agencé un outil (5) constitué d'au moins deux pièces de mâchoire (5a, 5b), la tige de traction/poussée (4) pouvant, pour l'ouverture et la fermeture d'au moins une pièce de mâchoire (5b) de l'outil (5), être couplée à au moins une branche de préhension pivotante (1) de la poignée de manoeuvre (2), et le corps allongé creux (3) et la poignée de manoeuvre (2) pouvant être reliés l'un à l'autre de manière amovible par l'intermédiaire d'un mécanisme de couplage, ce mécanisme de couplage étant réalisé en tant que dispositif combiné de serrage et d'encliquetage, l'extrémité proximale du corps allongé creux (3) étant de configuration conique pour la réalisation du dispositif de serrage, et un alésage de réception (15), ouvert côté distal et s'étendant dans la direction axiale, étant formé dans la poignée de manoeuvre (2) et destiné à recevoir l'extrémité proximale du corps allongé creux (3), l'alésage de réception (15) présentant un cône conjugué se rétrécissant de manière correspondante,
**caractérisé en ce que** le cône conjugué pour la réception de l'extrémité proximale du corps allongé creux (3), est réalisé dans une douille (16) montée coulissante en direction axiale dans l'alésage de réception (15), la douille (16) étant montée axialement coulissante dans l'alésage de réception (15) à l'encontre de la force d'un élément de ressort (18), et la douille (16) étant précontrainte, par l'intermédiaire de l'élément de ressort (18), dans la position de montage libérant le couplage entre le corps allongé creux (3) et la poignée de manoeuvre (2).

2. Instrument médical à corps allongé tubulaire selon la revendication 1, **caractérisé en ce que** pour la réalisation du dispositif d'encliquetage, un élément d'encliquetage (17), qui peut être déplacé sensiblement de manière transversale à l'alésage de réception (15), est agencé dans la poignée de manoeuvre (2).

3. Instrument médical à corps allongé tubulaire selon la revendication 2, **caractérisé en ce que** l'élément d'encliquetage (17) est constitué d'un organe de verrouillage (17a) ainsi que d'un bouton de déblocage (17c) couplé avec l'organe de verrouillage (17a).

4. Instrument médical à corps allongé tubulaire selon la revendication 2 ou la revendication 3, **caractérisé en ce que** l'élément d'encliquetage (17) est précontraint par l'intermédiaire d'un élément de ressort (19) en direction de la position d'encliquetage.

5. Instrument médical à corps allongé tubulaire selon l'une des revendications 2 à 4, **caractérisé en ce que** dans l'extrémité proximale du corps allongé creux (3) est formé au moins un évidement (20) destiné à recevoir par enclenchement l'élément d'encliquetage (17).

6. Instrument médical à corps allongé tubulaire selon la revendication 5, **caractérisé en ce qu'**aussi bien dans l'extrémité proximale du corps allongé creux (3) que dans la douille (16), est formé au moins un évidement (20) destiné à recevoir par enclenchement l'élément d'encliquetage (17).

7. Instrument médical à corps allongé tubulaire selon l'une des revendications 1 à 6, **caractérisé en ce que** dans la poignée de manoeuvre (2) est agencé au moins un élément de guidage (21) par l'intermédiaire duquel l'extrémité proximale du corps allongé creux (3) peut être orientée dans l'alésage de réception (15).

8. Instrument médical à corps allongé tubulaire selon la revendication 7, **caractérisé en ce que** l'élément de guidage (21) est réalisé sous forme de vis sans tête.

9. Instrument médical à corps allongé tubulaire selon la revendication 7 ou la revendication 8, **caractérisé en ce qu'**à l'extrémité proximale du corps allongé creux (3) est formée au moins une rainure de guidage (22) pour recevoir l'élément de guidage (21).

10. Instrument médical à corps allongé tubulaire selon la revendication 9, **caractérisé en ce qu'**à l'extrémité proximale du corps allongé creux (3) sont formées deux rainures de guidage (22) mutuellement opposées, pour recevoir l'élément de guidage (21).

11. Instrument médical à corps allongé tubulaire selon l'une des revendications 7 à 10, **caractérisé en ce que** l'élément de guidage (21) forme une butée pour la douille (16).
